# EUROPEAN PATENT APPLICATION

(11) **EP 4 741 054 A1**
(43) Date of publication of application: **13.05.2026**
(21) Application number: 25214481.1
(22) Date of filing: 08.11.2025
(51) Int. Cl.: B01J 29/65, B01J 29/68, B01J 29/76, C01B 39/02, C07C 51/15, C07C 53/08, C07C 67/00, C07C 69/14

(54) **A CATALYST FOR THE PRODUCTION OF ACETIC ACID AND METHYL ACETATE FROM CO2 AND CH4 BASED ON ZEOLITES WITH CONTROLLED DISTRIBUTION OF ALUMINUM ATOMS**

(30) Priority: 08.11.2024 PL 45023424
(71) Applicant: UNIWERSYTET JAGIELLONSKI, 31-007 Kraków (PL); Czech Academy of Sciences, 182 23 Prague (CZ)
(72) Inventor: Tarach, Karolina, 30-389 Kraków (PL); Góra-Marek, Kinga, 31-236 Kraków (PL); Sobalska, Julia, 32-049 Przeginia (PL); Tabor, Edyta, 182 23 Prague (CZ); Dedecek, Jiri, 182 23 Prague (CZ); Mlekodaj, Kinga, 182 23 Prague (CZ); Kaucký, Dalibor, 182 23 Prague (CZ)
(74) Representative: Witek, Rafal

(57) **Abstract**

A catalyst based on zeolite with a controlled distribution of aluminium atoms in the zeolite structure and with a specific location of divalent transition metal cations, such as iron and zinc cations, in the zeolite and a method for producing acetic acid and methyl acetate from carbon dioxide and methane using such a catalyst are disclosed. The structure of the zeolite is selected from CHA, FER, MOR and *BEA.

## Description

The object of the invention is a method for obtaining a zeolite-based catalyst with a controlled distribution of aluminum atoms, ensuring the desired location of transition metal cations, and the use of such a catalyst for the conversion of methane and carbon dioxide into acetic acid and methyl acetate.

Carbon dioxide and methane are gases that significantly contribute to the greenhouse effect. High concentrations of CO₂ in the atmosphere have a direct impact on global warming and ocean acidification. Therefore, the transformation of CO₂ into chemical compounds that can be further used as valuable substrates in the chemical industry is an ideal solution to reduce the amount of CO₂ in the environment. Methane, the main component of shale gas, is a promising starting material for the production of fuels and chemicals.¹⁻⁴ Therefore, using CO₂ and CH₄ as raw materials for the production of value-added chemicals is an attractive method for efficiently utilizing shale gas while simultaneously reducing atmospheric CO₂ concentrations.

Acetic acid is a crucial product for the food industry and an important intermediate for many commercial chemicals, such as acetic anhydride, vinyl acetate, and alkyl acetates. It can also be used as a solvent in the synthesis of terephthalic acid.⁵⁻⁷ It is one of the most important industrial chemicals used in the manufacture of volatile organic esters, such as ethyl and butyl acetates, vinyl acetate, cellulose acetate, and metal acetates. The growing use of acetic acid in terephthalic acid production is expected to support market expansion. In the manufacture of polyester resins, which are widely used in polyester films, PET resins, and polyester fibers, terephthalic acid plays a key role as a building block component. Terephthalic acid is also used in the fabrication of textiles. Vinyl acetate monomers, produced from acetic acid, are often used to make resins and polymers used in textiles, paints, coatings, adhesives, and films.

Many technological processes for obtaining acetic acid have been developed based on various reactions, including: (i) partial oxidation of CH₄ with oxygen and subsequent reaction with carbon monoxide,⁸⁻¹⁰ (ii) selective oxidation of ethane with oxygen,^{11,12} (iii) oxidation of ethanol,¹³⁻¹⁵ or (iv) carbonylation of methanol¹⁶⁻²⁰ and fermentation.²¹ Among these processes, carbonylation of methanol is the most widely used in industry. The industrial synthesis of acetic acid began with the BASF process and was later improved by the Monsanto process²² and the Cativa process.²³ The BASF process of methanol carbonylation used high pressure (approximately 68 MPa) and a cobalt-based catalytic system promoted by iodide ions. The Monsanto process used a homogeneous Rh catalyst, which enabled the synthesis of acetic acid with high selectivity (>99%) at a pressure of approximately 3-6 MPa and a temperature of 150-200 °C. The Cativa process was developed using a more active Ir-based catalyst,^{24,25} which resulted in fewer side products.

The optimal solution is therefore the direct conversion of CH₄ and CO₂ into acetic acid, a process that is environmentally friendly and aligns with the principles of green chemistry. However, the direct transformation of methane and carbon dioxide into acetic acid is thermodynamically unfavorable; hence, the use of a catalyst is necessary. For example, Taniguchi and co-workers²⁶ studied the reaction between methane and carbon dioxide using a vanadium catalyst in a solution containing trifluoroacetic acid (TFA) and peroxydisulfate (as an oxidant). The yield of this reaction was 15.7% based on CH₄. The study showed that the reaction yield was independent of CO₂ pressure and that the reaction proceeded without the participation of CO₂. This suggests that acetic acid was not formed by the reaction between CO₂ and CH₄. Huang and co-workers²⁷ employed a periodic method for obtaining acetic acid using a Co-Cu Cu/Co₅ catalyst; however, the composition and properties of the catalyst were not precisely specified. Initially, the catalyst was exposed to methane, which, according to the authors, generates a CHx group on the surface; then, the gas flow was changed to CO₂. This process was repeated several times. Apart from acetic acid, other products were formed (alcohols, ketones, formic acid, and cyclopentane derivatives), and the reaction yield was not reported. The selectivity toward acetic acid was 21%. Esther M. Wilcox and co-workers²⁸ monitored the interaction between CO₂ and CH₄ over 5% Pt/Al₂O₃ and 5% Pd/carbon catalysts. Studies using FTIR spectroscopy and temperature-programmed reduction confirmed the formation of acetic acid from a mixture of CO₂ and CH₄ at a temperature of approximately 400 °C.

In turn, the patent by Freund and Wambach²⁹ claims a method for the direct synthesis of acetic acid from CO₂ and CH₄ using a solid catalyst described as containing "one or more metals from groups VIA, VIIA, VIIIA" supported on alumina, aluminum hydroxide, or silicon dioxide. The reaction conditions are in the temperature range of 100-600 °C and pressure range of 0.1-20 MPa. According to the patent, under these conditions, selectivity of 70-95% and an unspecified "sufficient conversion on a commercial scale" can be achieved. The patent does not provide any actual data or experimental examples.

Zeolites are among the most common industrial catalysts and supports. These materials exhibit high thermal stability, and upon the introduction of transition metal ions into their structure, function as active and selective catalysts for redox processes. The possibility of direct transformation of CO₂ and CH₄ into acetic acid has been confirmed by theoretical calculations, which demonstrate the potential of activation of CO₂ and CH₄ on metal centers in zeolites.^{30,31} In the case of acetic acid formation over zeolitic catalysts, primarily zeolites with MFI and MOR topologies modified with copper or zinc ions have been used.

Catalytic systems based on M-ZSM-5 zeolites (where M is Li⁺, Na⁺, K⁺, and Ca²⁺) further modified with copper ions, were active in the formation of acetic acid from carbon dioxide and methane in the temperature range of 425-525 °C. However, these catalysts were rapidly deactivated due to copper aggregation under reaction conditions, resulting in a decrease in the number of active sites.³²

Experimentally, the direct transformation of CH₄ and CO₂ to acetic acid over Zn-ZSM-5 zeolite was confirmed exclusively by NMR spectroscopy. The transformation was observed within a temperature range of 250-500 °C.³³ To date, no studies have reported the formation of acetic acid from CO₂ and CH₄ over zinc catalysts based on zeolitic structures other than ZSM-5 (MFI topology), nor on any zeolites containing iron cations in their structure.

The speciation of transition metal cation species acting as redox centers, and thus as active centers generating the expected catalytic activity, is crucial for determining catalyst parameters.

Speciation, in the qualitative sense, refers to the distribution of an element among its various chemical forms including isolated cations (M⁺), neutral or charged metal (or oxide) nanoclusters composed of a defined number of atoms (Mₙ or M⁺ₙ), amorphous metal (or oxide) nanoparticles, or crystalline forms of the metallic (or oxide) phase. These distinct speciation states exhibit different catalytic activity. Current research is primarily exploratory, focusing on the nature of active sites, theoretical studies, and the detection of CO₂ and CH₄ reaction products using spectroscopic methods. In the present application, the results of operando and in situ spectroscopic studies are presented to define the dependence of the catalyst surface properties on its activity and selectivity.

Such studies are carried out only sporadically, as the high temperatures involved adversely affects the reliability of spectroscopic measurements. It should be emphasized that the operando and in situ methodology - combining rapid scan IR spectroscopy with mass spectrometry - is the only one approach reported to the date for investigating this process. Its successful application was enabled by relatively low temperature range in which the process was examined.

The design of a zeolitic catalyst incorporating redox centers for a targeted process necessitates precise identification of the active site types responsible for the reaction, elucidation of the methods for their generation, and controlled introduction of these sites in the required quantities. Therefore, full control over the speciation of transition-metal species - including their concentration - is essential. Cationic species with valence states exceeding those stabilized by the zeolite framework tend to undergo hydrolysis readily, leading to transformations in their speciation and, frequently, changes in dispersion. Consequently, controlling the physicochemical properties of the zeolite support itself becomes the decisive factor governing the stability, speciation, and ultimately the catalytic performance of the introduced metal species. This is realized by incorporating aluminum atoms into the zeolite framework in configurations that stabilize the divalent ion as an isolated site, thereby preventing undesirable hydrolysis process. Moreover, ensuring the close proximity of cooperating transition-metal cations is essential, a requirement that is likewise met through the preferential and targeted placement of aluminum atoms within the framework. Determination of the aluminum-atom distribution in zeolites - both in commercially available materials such as ferrierite and in those synthesized in the Inventors' laboratory, including *BEA and chabazite (e.g., SSZ-13, the high-silica form of CHA with Si/Al > 6) - is feasible only through the use of cobalt ions (Co²⁺). As divalent cations, Co²⁺ species preferentially occupy zeolitic rings that contain two aluminum atoms, thereby enabling precise identification of their location. This method for quantifying the existence of two aluminum atoms within a single zeolitic ring is crutial for determining the concentration of other divalent transition metal cations (Fe²⁺, Cu²⁺, Zn²⁺, Co²⁺), which can be stabilized exclusively in such sites. Accordingly, the development of synthesis strategies that promote the preferential location of aluminum atoms - and thus the selective stabilization of divalent ions (Co²⁺, Cu²⁺, Fe²⁺, Zn²⁺) - is critical for generating a high proportion of catalytically active redox centers, i.e., isolated divalent cations embedded within the zeolitic framework. Furthermore, for zeolites with topologies (CHA Si/Al > 5) and *BEA, a dedicated synthesis method has been developed^{34,35,36} that guarantees the preparation of materials with a high concentration of aluminum atoms in the framework (Si/Al ≤ 5). This invention employs a novel strategy that integrates fundamental understanding of aluminum-atom placement in zeolitic matrices, deliberate enhancement of their concentration through low Si/Al, and the controlled stabilization of catalytically active transition-metal species. Particular emphasis is placed on stabilizing cations in close proximity -specifically binuclear cation centers located at a distance of approximately 8 Å - which exhibit high catalytic activity in the activation of reactants and subsequent desorption of desired products. The unique catalytic properties of binuclear transition metal centers in zeolites in the oxidation of methane to methanol with molecular oxygen are the object of an international patent application³⁷ and have also been extensively documented in the scientific literature.^{38,39,40-44}

The international patent application WO2015/197036A1 discloses a catalyst for the selective catalytic reduction of nitrogen oxides, characterized in that it comprises a beta zeolite with an aluminum content in the lattice corresponding to a Si/Al ratio in the range of 3 to 8 and containing cobalt ions as active sites.

International patent application WO2020/200336 discloses a catalyst for the production of methanol from methane. The catalyst comprises a zeolite in which aluminum atoms are arranged in pairs within the framework, wherein the content of such aluminum pairs is at least 10% relative to the total number of aluminum atoms in the zeolite. The catalyst further comprises a transition-metal cation M coordinated at β-cation positions, wherein M is selected from the group consisting of V, Cr, Mn, Fe, Co, Ni, Cu, and Ag. The molar ratio of transition metal M to Al is within the range of 0.01 to 0.5, and the Si/Al ratio is in the range of 2 to 9. The disclosure specifies that the zeolite is not ZSM-5 or mordenite. The international patent application WO2020/200336 discloses a catalyst for the production of methanol from methane. The catalyst comprising a zeolite in which aluminum atoms arranged in pairs within the framework, wherein the content of such aluminum pairs is at least 10% relative to the total number of aluminum atoms in the zeolite. The catalyst further further comprises a transition-metal cation M coordinated at beta(β)-cation positions, wherein M is selected from the group consisting of V, Cr, Mn, Fe, Co, Ni, Cu, Ag. The molar ratio of transition metal M to Al is within in the range of 0.01 to 0.5;
and the Si/Al ratio is in the range of 2 to 9. The disclosure specifies that the zeolite is not ZSM-5 and mordenite.

The object of the invention is to provide an industrial method for producing acetic acid and methyl acetate from CO₂ and CH₄, and, in particular, a catalyst suitable for this transformation. It is particularly desirable that the reaction be carried out at atmospheric pressure and at temperatures not exceeding 500 °C.

It is desirable that the proposed method ensure a yield of at least 20%.

The object of the invention is a catalyst for the production of acetic acid and methyl acetate from methane and carbon dioxide, characterized in that it comprises a zeolite containing:
- Al atoms arranged in pairs, wherein each pair is represented by two Al atoms located in one zeolite ring,
- divalent transition metal M cations, preferably Fe and Zn,

wherein the ratio of transition metal M to Al is in the range of 0.06 to 0.45, while the weight content of transition metal M is less than 10% by weight, and the Si/Al ratio in the zeolite is in the range of 2.5 to 10,
wherein the zeolite has a structure selected from CHA, FER, MOR, or *BEA structures, as determined according to the Nickel-Strunz classification of zeolite structures.

Another object of the invention is a method for producing acetic acid and methyl acetate, characterized in that:
a) water and ammonium ions are removed from the catalyst, preferably by heating it more than 30 minutes at a temperature above 300 °C, wherein a catalyst according to the invention as defined above is used;
b) the catalyst is contacted with methane and subsequently with carbon dioxide (or in the reverse order, or with a mixture) at temperatures below 450 °C under conditions of at least atmospheric pressure (1 atm.).

Preferably, in step a), heating is carried out in a vacuum or in a stream of an inert gas (e.g., helium, nitrogen).

Preferably, in step a), heating is carried out at a temperature of at least 500 °C.

To better explain the invention, the description is complemented by the attached Figures.
Figure 1 shows the FT-IR spectra of FER zeolite in the region of framework vibrations, illustrating the presence of bands in the 960-900 cm⁻¹ range, thereby confirming the incorporation of iron and zinc ions at the ion-exchange cationic sites of the FER zeolite framework.
Figure 2 shows FT-IR spectra recorded for zeolites containing Fe²⁺ ions introduced via ion-exchange into zeolites with controlled organization of aluminum atom : CO sorption (A) and NO sorption (B).
Figure 3 shows the difference IR spectra recorded at room temperature for the Fe-*BEA, Fe-CHA, Fe-FER, and Zn-FER catalysts following CO₂ adsorption (a), methane adsorption (b), and heating the system to 250 °C and cooling to room temperature (c).
Figure 4 shows the rapid scan IR spectra (A) recorded in the temperature range of 250-300 °C for the Zn-FER, Fe-*BEA, and Fe-FER catalysts after adsorption of a mixture of CH₄ and CO₂, with the evolution of the signal at m/z=60 (acetic acid) (B). Synchronous 2D COS maps for the studied process (C).
Figure 5 shows MS signals of acetic acid (m/z=60) (A) and methyl acetate (m/z=74) (B) after adsorption of an equimolar mixture of CH₄ and CO₂ on the tested catalysts (Zn-FER, Fe-FER, Fe-*BEA, and Fe-CHA).

### Example 1. Preparation of the catalyst.

### Synthesis of zeolites with the desired distribution of aluminum atoms in the framework

### A. Verified synthesis of CHA zeolite

12.06 g of KOH (Sigma-Aldrich) was dissolved in 213 g of distilled water, after which 25 g of commercially available zeolite Y with a Si/Al ratio of 2.6 (CBV500, Zeolyst International) was added, and the mixture was shaken for 30 seconds. The vessel was then transferred to an oven for crystallization under static conditions (without stirring, at 95 °C, 4 days). Following crystallization, the product was filtered, washed with distilled water, and air-dried at room temperature. The obtained zeolite exhibited structural characteristics typical of the CHA topology and high aluminum content, i.e., Si/Al = 2.4.⁴⁸

The second method for synthesizing CHA zeolites employed in this application was based on a previously patented procedure.⁴⁹ This method involves the interzeolite conversion of zeolites X, A, or ZSM-5 (serving as the aluminum source) into a zeolite with the CHA topology. The advantage of this method is the ability to control both the aluminum atom content (from high-aluminum preparations to high-silica materials with Si/Al > 12, zeolite SSZ-13) and the spatial distribution of aluminum atoms in the CHA structure of the resulting zeolitic material. This approach is innovative because, as mentioned earlier, it enables the introduction of catalytically active transition metal centers in varying concentrations and forms (dispersed cations, cooperating binuclear centers, dimers, oligomers). It is advantageous to use a zeolite with a tailored distribution of aluminum atoms as a catalyst for the production of acetic acid from CO₂ and CH₄, which is directly related to the targeted location of redox active centers, i.e. zinc and iron centers.

### B. Synthesis of CHA zeolite using Y zeolite as a source of aluminum atoms

100 g of a sodium silicate solution containing 26.5 wt.% SiO₂ was added to 240 g of distilled water and stirred for 15 min. Then, 6 g of Na-Y zeolite (Si/Al = 2.8), which was the sole source of Al atoms, was added to the mixture and stirred for 30 min. Then, 52.6 g of a 40 wt.% solution of N, N, N-trimethyl-1-adamantylammonium hydroxide was added, and the reaction mixture was stirred for an additional 30 min before being transferred to an autoclave. Crystallization of the zeolite with the CHA structure (SSZ-13) was carried out at 140 °C for 6 days with continuous rotation of the autoclave. After synthesis, the solid phase was filtered, washed with distilled water, dried at 77 °C, and then calcined at 500 °C (at a heating rate of 1 °C/min) in air flow for 24 hours. The resulting CHA zeolite had a Si/Al ratio of 7. It should be noted that using CHA instead of Y zeolite in the synthesis method described above **led to unusual and desired Si/Al ratios.** For example, the use of:
- zeolite X resulted in CHA zeolite with a Si/Al ratio of 5.2
- zeolite A resulted in CHA zeolite with a Si/Al ratio of 5.6
- zeolite ZSM-5 resulted in CHA zeolite with a Si/Al ratio of 12.9
- zeolite USY resulted in CHA zeolite with a Si/Al ratio of 16.

The advantage of the above-described method for obtaining CHA-type zeolite lies in the use of zeolites with different topologies as a source of aluminum atoms. This renders this synthesis method highly versatile and allows obtaining the preparation of zeolites with a wide range of Si/Al ratio and large diversity of aluminum distribution in the framework.⁵⁰

The analysis of the aluminum atom arrangement using cobalt cations as probes for the locating of aluminum pairs confirmed that more than 60% of aluminum atoms occupy positions in the CHA zeolite framework that enable the stabilization of transition metals within the zeolite matrix. These metals are stabilized in forms (dispersed cations, cooperating binuclear centers, dimers, oligomers) that can serve catalytically active centers.

The preparation of catalysts for CO₂ and CH₄ conversion based on the aluminum-rich zeolite *BEA was carried out according to a known method for synthesizing this zeolite without the use of an organic template.^{36,51} Template-free zeolite synthesis provides significant environmental and economic advantages. Moreover, obtaining a material with a high aluminum content in the framework enables the stabilization of a substantial fraction of transition metal centers exhibiting catalytic properties in the activation of CO₂/CH₄ and the production of acetic acid.

### C. Synthesis of aluminum-rich *BEA zeolite

The synthesis of aluminum-rich *BEA zeolite was carried out using a synthesis gel with a molar composition of 1 Si : 0.09 Al : 0.6 NaOH : 25 H₂O and 5-20 wt.% of silica-rich *BEA zeolite seeds. The gel was prepared as follows: an Al source was added to an aqueous NaOH solution and stirred for 30 minutes at room temperature. Colloidal silica was then added, and the mixture was stirred for 3 hours. Subsequently, the silica-rich *BEA zeolite was added to the suspension and stirred for 30 minutes. The prepared gel was transferred to an autoclave and stirred (anchor stirrer, 30 rpm) at 120 °C for 120 hours or 140 °C for 50 hours. The autoclave was then cooled to 90 °C and depressurized. The solid product was filtered, washed thoroughly with distilled water, and dried overnight at 80 °C.

### Generation of redox-active centers for the conversion of CO₂ and CH₄ into acetic acid and methyl acetate

### A. Introduction of iron cations into FER, CHA, and *BEA zeolites

Iron cations were introduced into FER, CHA, and *BEA zeolites using a previously patented method based on zeolite impregnation with a solution of iron chloride in acetylacetone.^{52,53} As the data will demonstrate, the catalytic formation of acetic acid (from CO₂ and CH₄) strongly depends on the distribution of aluminum atoms in the zeolite framework. Iron species located in zeolites containing only one aluminum atom in the zeolite ring are inactive. In contrast, the formation of catalytically active iron centers, capable of promoting the transformation of CO₂ and CH₄, requires precise localization of iron cations and their stabilization within rings containing two aluminum atoms.

Therefore, it is necessary to determine the distribution of aluminum atoms in the tested zeolite matrices and then subsequently calculate the appropriate FeCl₃ concentration in acetylacetone to ensure that the iron ions to occupy the cationic positions. The range of catalytically active Fe-FER, Fe-CHA, and Fe-*BEA samples corresponds to Fe/Al molar ratios from 0.01 to 0.4. The first step in the preparation of iron catalysts for acetic acid production is to obtain the ammonium form of zeolites by three-fold ion exchange of FER, CHA, or *BEA zeolites with 1M ammonium nitrate solution (100 ml of ammonium nitrate solution was used per 1 g of zeolite). Subsequently, NH₄-FER, NH₄-CHA, or NH₄-*BEA zeolites were granulated (fraction of 0.3-0.6 mm), dried for 4 hours at 120 °C, and impregnated with a solution of FeCl₃ in acetylacetone (at a concentration ensuring the desired concentration of iron centers) for 12 hours. The FeCl₃-impregnated zeolite was then transferred onto filter paper to allow the acetylacetone to evaporate. In the next step, the sample was subjected to vacuum for 1 hour at 100 °C and then at 350 °C for 3 hours. After cooling, the sample was washed with distilled water, filtered, and dried at room temperature. In the final step of Fe-zeolites preparation, the organic content was removed by calcination in an air stream at 450 °C for 16 h. The advantage of the above-described method for preparing iron zeolites lies in producing materials in which iron ions occupy specific cationic positions within the zeolite. This arrangement enables the formation of cooperative binuclear centers, spaced approximately 6-9 Å apart, which are highly effective in activating reactants and exhibit excellent catalytic activity for the conversion of methane and carbon dioxide to acetic acid.^{38,39,40-44}

It is worth mentioning that catalytic activity in the studied systems can only be demonstrated by those iron centers that are capable of carrying out the redox cycle by activating the CO₂ and/or CH₄ molecules. The presence of high-aluminum zeolites (CHA and *BEA) enables the formation of high concentrations of iron centers, which can translate into higher acetic acid production efficiency. In the case of high-silica forms of CHA, i.e., SSZ-13, it is possible to stabilize iron cations at several cationic positions, whose geometry and physicochemical properties³⁴ enable reagent activation.

### B. Introduction of zinc ions

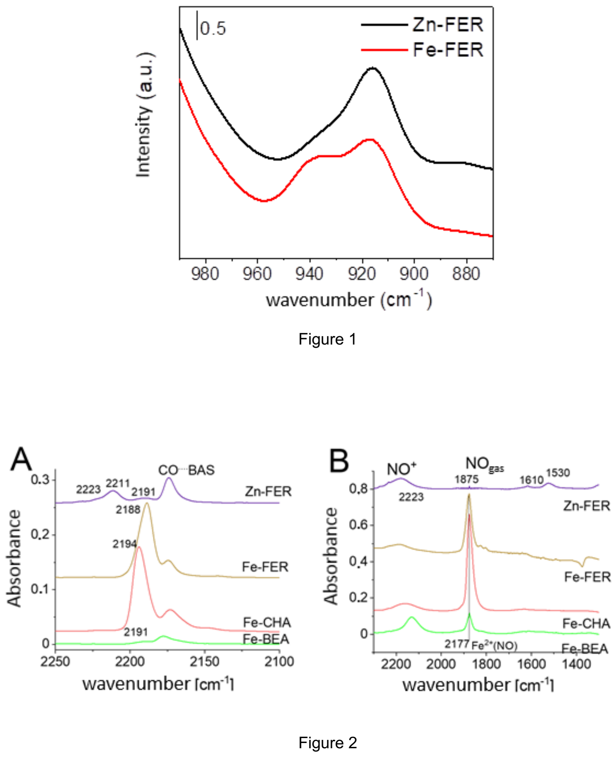

Zinc forms of FER, CHA, and *BEA zeolites, defined by Zn/Al ratios ranging from 0.01 to 0.4, were prepared by ion exchange. The first step in obtaining zinc catalysts for acetic acid production was to obtain the ammonium form of zeolites by three-fold ion exchange of FER, CHA, or *BEA zeolites with 1M ammonium nitrate solution (100 ml of ammonium nitrate solution was used per 1 g of zeolite). Subsequently, the ammonium forms of FER, CHA, and *BEA were subjected to further ion exchange with aqueous solutions of zinc nitrate (Zn(NO₃)₂ x 6 H₂O) with a Zn concentration of 0.001-0.05 mol/L at room temperature (1-5 x 16 h, 100 ml of solution per 1 g of zeolite). Obtaining the maximum zinc concentration in FER, CHA, and *BEA required the use of the zinc acetate impregnation method. The NH₄-forms of FER, CHA, and *BEA zeolites were sodium-exchanged with 1 M sodium nitrate solution (three-fold exchange; 24 h, at room temperature; 100 mL of solution per 1 g of zeolite). Na-FER, -CHA or -*BEA zeolites were zinc-exchanged with an aqueous solution of zinc acetate (ZnC₄H₆O₄) with a Zn concentration of 0.05 mol/I at room temperature (3 x 16 hours, 100 ml of solution per 1 g of zeolite). After washing the obtained preparations with distilled water, the samples were air-dried and then calcined in an air flow at 450 °C for 16 hours. Figure 1 shows example of FTIR spectra recorded for FER zeolite in the T-O-T framework vibration range, documenting the presence of bands in the frequency range of 960-900 cm⁻¹, thus confirming the introduction of iron and zinc ions into the FER matrix.

Zinc centers incorporated into zeolite matrices may exhibit varying valence, symmetry, and structure (atomically dispersed, monomers, dimers, binuclear centers, oxo-centers, bridging bonds, oxides), which is reflected in their catalytic properties. Zinc centers are a key element in the activation of methane, which, in the next step, can react with activated CO₂, or the CO₂ molecule will be incorporated into the methyl group stabilized on the zinc cation (Zn-CH₃). In the discussed process, zinc centers that are capable of activating reagent molecules are desirable, so binuclear centers are most effective in this process, the concentration of which is modulated by the synthesis of zeolite matrices with the desired location of aluminum atoms.

### Example 2. A method for producing acetic acid and methyl acetate using a catalyst according to the invention

The catalytic reaction products were monitored using mass spectrometry. The catalyst, in the form of a compressed disc, was placed in a quartz reactor. The reactor temperature was controlled by a thermocouple. A 0.02 to 0.05 g sample of the catalyst was used for the test. Before the reaction, the catalyst sample was heated in a helium flow (rate of 30 ml/min) at 500 °C for 1 hour. The temperature was then decreased (at the rate of 10 °C/min) under an inert gas flow to 25 °C. Subsequently, the sample was exposed to a gas mixture of CO₂ (15 ml/min) and CH₄ (15 ml/min), and the temperature was gradually increased at 5 °C/min up to 500 °C. The reaction products leaving the reactor were continuously monitored throughout the experiment. The same reaction was performed in a batch-type reactor in the 25 - 450 °C temperature range, where the catalysts were contacted with a 1:1 mixture of CH₄ and CO₂, and the formation of products was monitored by mass spectrometry. Signals were detected at m/z = 60 (acetic acid) and 74 (methyl acetate). The m/z = 60 signal was integrated and compared with calibration data for acetic acid to determine the acetic acid production yield. In tests conducted using the catalyst according to the invention, the yield was always greater than 20%.

### Example 3. Spectroscopic studies of the conversion process of CO₂ and CH₄ into acetic acid and methyl acetate

The speciation of transition metal cation species, which act as redox centers and thus serve as active sites in generating the expected catalytic activity, was investigated using IR spectrometry. Figure 2 presents the spectra of adsorbed CO, documenting the presence of Fe²⁺ isolated cations in the form of Fe²⁺(CO) in the Fe-FER, Fe-CHA, and Fe-*BEA catalysts, and Zn²⁺ ions in Zn-FER. Differences in band position and intensity indicate differences in the binding strength of CO to the cationic centers (Fe²⁺) and their varying concentrations, ruled by the Si/Al ratio in the zeolite framework and the intentionally introduced metal content. The strongest Fe²⁺ sites are present in the Zn-FER zeolite. In the case of Fe-zeolites, the lower wavenumber of the monocarbonyl bands is ruled by the significant contribution of the backdonation effect, which is absent in the case of Zn²⁺ ions. Sorption of nitric oxide(ll) over the tested zeolites also differentiates the strength of interaction of the NO molecule with the redox centers present in the zeolites. The formation of mononitrosyl bands (bands around 2177 cm⁻¹) is observed only for the iron samples. In the case of Zn²⁺ ions, no interaction with the NO molecule is observed. However, the presence of nitrate species (1610 and 1530 cm⁻¹) indicates the involvement of Zn²⁺ in the reaction with NO.

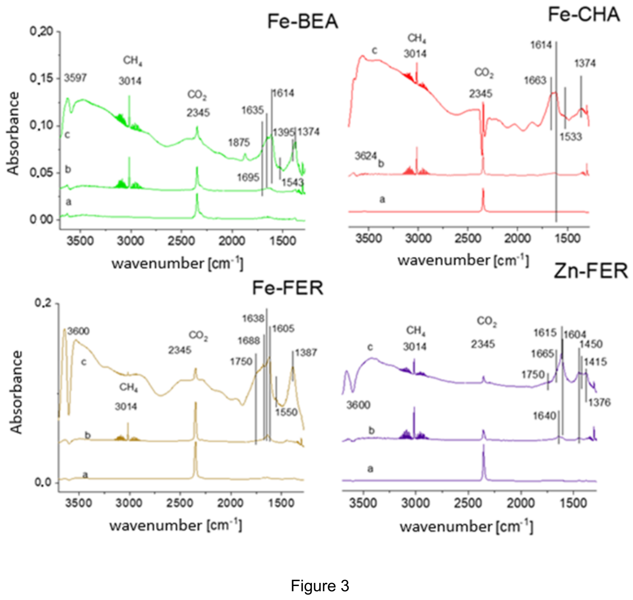

The influence of the unique location of cationic centers on the activation of methane and carbon dioxide molecules was confirmed in FT-IR spectroscopic studies (Figure 3) carried out for *BEA, FER, and CHA zeolites. For comparison, the same measurements were performed for a sample containing only proton centers (native sample). The reaction products were also identified spectroscopically (IR spectroscopy).

For this purpose, each product was individually adsorbed onto the catalyst surface as the sole reactant, and its diagnostic bands were recorded. These characteristic bands were then used to unambiguously identify the corresponding species within the reaction mixture.. The catalytic performance of these materials was evaluated in the conversion of CO₂ and CH₄ to acetic acid and methyl acetate. The formation of these products was confirmed by studies carried out under in situ conditions using complementary techniques: transmission IR spectroscopy, which identified diagnostic bands characteristic of the reactants, and mass spectrometry, which monitored the time- and temperature-dependent evolution of the corresponding m/z signals for the reaction products exiting the spectroscopic reactor. The catalysts described in this application operate at low temperatures; acetic acid was identified as a product of the methane and CO₂ reaction already at 150 °C. The presented FTIR spectroscopy results refer to the process at 150 °C and 200 °C using CH₄ and CO₂ as pure gases in stoichiometric composition. The beneficial effect of the presence of Zn²⁺ and Fe²⁺ ions was confirmed in the studies of catalytic conversion of CH₄ and CO₂ (Figure 3). After introducing methane into the catalyst/CO₂ system, the appearance of IR bands in the wavenumber range of 1750-1300 cm⁻¹ was observed for all preparations at room temperature. Their presence is associated with the formation of chemical compounds containing the CH₃ group (1450-1374 cm⁻¹) and the -CHO or COO⁻ group (1750-1450 cm⁻¹). The formation of the aforementioned products is also related to the disappearance of the bands of the reaction substrates, i.e., methane (3014 cm⁻¹) and CO₂ (2345 cm⁻¹).

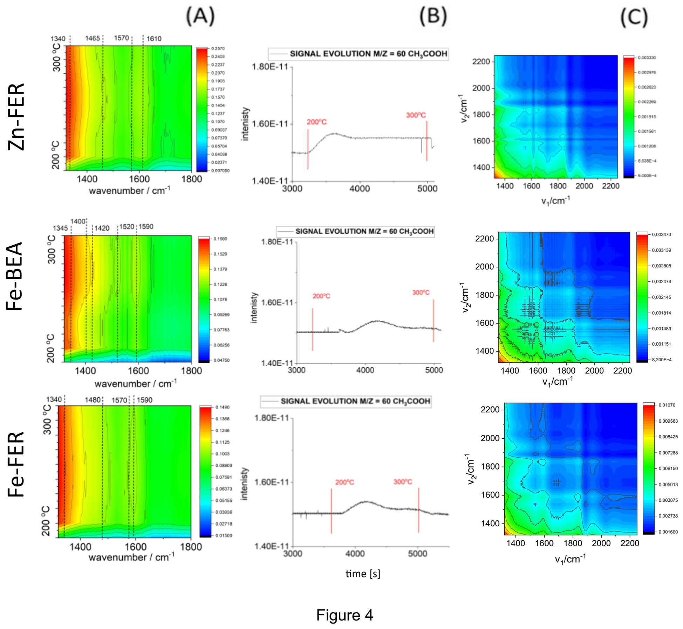

The products of the above-mentioned process were unambiguously identified using coupled rapid-scan IR spectroscopy (detection of reactants and products adsorbed on the catalyst surface) and mass spectrometry (detection of products released from the catalyst surface) (Figure 4). A 20 mg disc-shaped catalyst was placed in an IR cell and pre-activated by thermal treatment under vacuum at 500 °C for 30 min to remove adsorbed water and decompose the ammonium ions. The temperature was then reduced to 200 °C. Once stabilized, methane and carbon dioxide (5 Torr each) were introduced. The formation of acetic acid in the 200 - 300 °C temperature range was monitored by tracking the time-dependent signals at m/z = 60 (acetic acid) and 74 (methyl acetate). Changes in the concentrations of the reactants and other products (formaldehyde, CO and CO₂) were also monitored.

Simultaneously, adsorption species formed on the catalyst surface were monitored by recording rapid-scan IR spectra in transmission mode. To determine the efficiency of acetic acid formation, comparative experiments were performed in which acetic acid was adsorbed onto the tested catalysts and its subsequent desorption from the surface was monitored, in an equivalent manner, using mass spectrometry. The analysis of the IR spectra in the range of C=O stretching vibrations and C-H deformation vibrations (1800 cm⁻¹ - 1320 cm⁻¹), shown in Figure 4A, unambiguously indicates the accumulation of compounds containing carbonyl/carboxyl groups on the catalyst surface (increase in the intensity of bands in the range (1620-1560 cm⁻¹)).

At the same time, the carbonyl/carboxyl species contain a methyl group CH₃- in their structure,whose presence is confirmed by the appearance of a band corresponding to the symmetric stretching vibrations in the wavenumber range of 1350-1340 cm⁻¹. The formation of compounds capable of strong adsorption on the zeolite surface, even at temperatures above 250 °C, is further evidenced by a pronounced perturbation of zeolite-specific vibrational bands specific (so-called overtones) in the region of approximately 1800 cm⁻¹. It should be emphasized that at the process temperature, i.e., above 200 °C, the adsorption of water molecules on zeolites is negligible; therefore, the IR bands observed in the spectra correspond exclusively to acetate forms. Analysis of the m/z profiles (Figure 4B) specific to acetic acid and methyl acetate indicates the formation of these compounds in the presence of each catalyst. Information on the accumulation of acetate species is also included in the synchronous 2D COS RS IR correlation maps, documenting also the correlation between the formation of COO⁻ (1580 cm⁻¹) and CH₃ (1345 cm⁻¹) species (positive cross-correlation peaks in the maps) - (Figure 4C).

As demonstrated above, zeolitic catalysts with iron or zinc cations effectively convert CO₂ and CH₄ to acetic acid. However, the mechanism of this process varies and is related to the type of cation incorporated into the zeolite structure. In the case of zeolites with zinc cations, the activated molecule is methane, which is stabilized as CH₃• on zinc cations. In the next reaction step, CO₂ insertion leads to the formation of acetic acid. Furthermore, activation of a methane molecule on two zinc cations, forming a binuclear center and CO₂ insertion, can lead to the formation of methyl acetate. Acetic acid formation on iron catalysts likely occurs via CO₂ activation in the presence of binuclear centers, followed by interaction with a methane molecule, which subsequently leads to the formation of acetic acid.

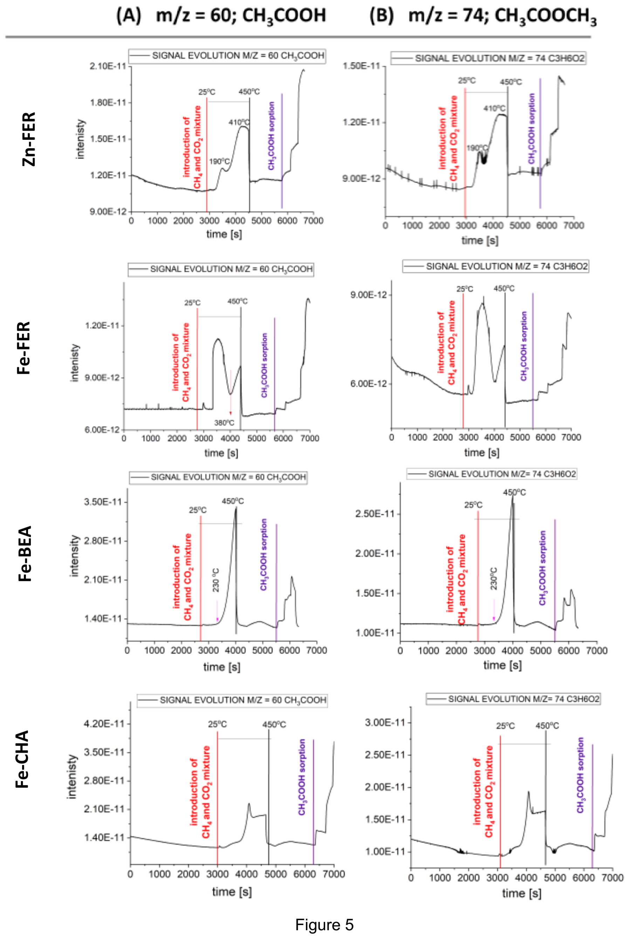

In addition, the production of acetic acid and methyl acetate was monitored in time-resolved mode using IR spectroscopy (observation of the catalyst surface state, i.e., detection of species adsorbed on the surface) and mass spectrometry (detection of products released from the catalyst surface) during a programmed temperature increase (Figure 5). A 20 mg disc-shaped catalyst was placed in an IR cell, and the catalyst was pre-activated by thermal treatment in a vacuum (500 °C, 30 min) to remove adsorbed water and decompose the ammonium ion. The temperature was then lowered to room temperature (25 °C). After the temperature stabilized, methane and carbon dioxide (5 Torr of each) were introduced. The formation of acetic acid in the temperature range of 25 - 450 °C was monitored by the time evolution of the signals at m/z = 60 (acetic acid) and = 74 (methyl acetate). Changes in the concentrations of the reaction substrates and other products (such as formaldehyde, CO, and CO₂) were also monitored. The results are presented in Figure 5.

In the presence of Zn-FER as a catalyst, production of acetic acid in a significant amount begins already at 150 °C, but the highest process efficiency occurs at temperatures above 220 °C (Figure 5A). From this temperature, the intensity of the acetic acid signal increases significantly up to 450 °C. Since the formation of acetic acid has been documented in the temperature range of 150 - 450 °C, observation of the process at higher temperatures is unnecessary. To determine the efficiency of the CO₂ and CH₄ transformation over zeolites into acetic acid, acetic acid was introduced into the reactor, and the temporal evolution of the m/z = 60 signal was monitored. Comparison of the m/z signals for acetic acid in both types of experiments showed the formation of acetic acid in the presence of the catalyst with efficiency ranging from 10-12% (at 190 °C) to 35% (in the temperature range of 220-450 °C). For Zn-FER, the formation of methyl acetate was observed concurrently with the production of acetic acid (Figure 5B), whereas methanol was generated only in the trace amounts.

Acetic acid production over the Fe-*BEA zeolite is at the same level as for the Zn-FER zeolite, i.e., 35%, but occurs at higher temperatures (above 230 °C with a maximum at 450 °C). Simultaneously, the formation of methyl acetate is observed with similar efficiency (signal at m/z = 74). When Fe-FER is employed as the catalyst, acetic acid is produced with an efficiency of about 40%. Simultaneously, the formation of methyl acetate is observed with approximately 30% efficiency (signal m/z = 74). When Fe-FER is used as a catalyst, methanol formation is not observed. On the other hand, the production of acetic acid in the presence of Fe-CHA as a catalyst proceeds with an efficiency similar to that observed for Zn-FER.

In summary, this application discloses , for the first time, zeolite-based catalysts modified by iron and zinc for the production of acetic acid and methyl acetate from CO₂ and CH₄.

These ions were introduced into the zeolite via ion exchange, with M/Al ratios ranging from 0.06 to 0.4 for all zeolites studied. Stabilization of zinc and iron cations in the zeolite structure was achieved through targeted zeolite synthesis, which controlled the distribution of aluminum atoms in the form of so-called aluminum pairs.

### Summary

In the work leading to the present invention, it was determined that the desired catalytic activity for the conversion of methane and carbon dioxide to acetic acid and methyl acetate could be achieved by:
(1) synthesizing zeolites with a controlled distribution of aluminum atoms,
(2) the use of the above-mentioned zeolites having a difined Si/Al ratio, which substantially increases the likelihood of stabilizing catalytically active divalent transition metal centers in ion-exchange positions, (3) incorporation of iron and zinc ions capable of activating CO₂ and CH₄ molecules into the aforementioned zeolites. Catalytic activity is a function not only of the specific location of the aluminum atoms, but also of the amount of cation introduced. It is worth noting that the proposed catalysts contain from 0.5% to 10% by weight of aluminum (M/Al = 0.06-0.4).
(4) efficient formation of acetic acid and methyl acetate was observed in the temperature range of 190-450 °C under atmospheric pressure (1 amt.).

### Literature

1. Tang, P.; Zhu, Q.; Wu, Z.; Ma, D., Methane activation: the past and future. Energy & Environmental Science 2014, 7 (8), 2580-2591.
2. Kondratenko, E. V.; Peppel, T.; Seeburg, D.; Kondratenko, V. A.; Kalevaru, N.; Martin, A.; Wohlrab, S., Methane conversion into different hydrocarbons or oxygenates: current status and future perspectives in catalyst development and reactor operation. Catalysis Science & Technology 2017, 7 (2), 366-381.
3. Paunović, V.; Zichittella, G.; Mitchell, S.; Hauert, R.; Pérez-Ramírez, J., Selective Methane Oxybromination over Nanostructured Ceria Catalysts. *ACS Catalysis* **2018,** *8* (1), 291-303.
4. Ding, S.; Hülsey, M. J.; Pérez-Ramírez, J.; Yan, N., Transforming Energy with Single-Atom Catalysts. Joule 2019, 3 (12), 2897-2929.
5. Yoneda, N.; Kusano, S.; Yasui, M.; Pujado, P.; Wilcher, S., Recent advances in processes and catalysts for the production of acetic acid. Applied Catalysis A: General 2001, 221 (1), 253-265.
6. Mei, Q.; Liu, H.; Shen, X.; Meng, Q.; Liu, H.; Xiang, J.; Han, B., Selective Utilization of the Methoxy Group in Lignin to Produce Acetic Acid. Angewandte Chemie International Edition 2017, 56 (47), 14868-14872.
7. Budiman, A.; Nam, J.; Park, J.; Mukti, R.; Chang, T.; Bae, J. W.; Choi, M., Review of Acetic Acid Synthesis from Various Feedstocks Through Different Catalytic Processes. Catalysis Surveys from Asia 2016, 20.
8. Shan, J.; Li, M.; Allard, L. F.; Lee, S.; Flytzani-Stephanopoulos, M., Mild oxidation of methane to methanol or acetic acid on supported isolated rhodium catalysts. Nature 2017, 551 (7682), 605-608.
9. Tang, Y.; Li, Y.; Fung, V.; Jiang, D.-e.; Huang, W.; Zhang, S.; Iwasawa, Y.; Sakata, T.; Nguyen, L.; Zhang, X.; Frenkel, A. I.; Tao, F., Single rhodium atoms anchored in micropores for efficient transformation of methane under mild conditions. Nature Communications 2018, 9 (1), 1231.
10. Moteki, T.; Tominaga, N.; Ogura, M., CO-Assisted Direct Methane Conversion into C1 and C2 Oxygenates over ZSM-5 Supported Transition and Platinum Group Metal Catalysts Using Oxygen as an Oxidant. ChemCatChem 2020, 12 (11), 2957-2961.
11. Jin, R.; Peng, M.; Li, A.; Deng, Y.; Jia, Z.; Huang, F.; Ling, Y.; Yang, F.; Fu, H.; Xie, J.; Han, X.; Xiao, D.; Jiang, Z.; Liu, H.; Ma, D., Low Temperature Oxidation of Ethane to Oxygenates by Oxygen over Iridium-Cluster Catalysts. Journal of the American Chemical Society 2019, 141 (48), 18921-18925.
12. Bordes-Richard, E., Application of concepts in heterogeneous oxidation of hydrocarbons: Mo, V-based oxide catalysts for oxidation of ethane and of n- and i-butanes. Catalysis Today 2021, 363, 1526.
13. Mostrou, S.; Nagl, A.; Ranocchiari, M.; Föttinger, K.; van Bokhoven, J. A., The catalytic and radical mechanism for ethanol oxidation to acetic acid. Chemical Communications 2019, 55 (79), 11833-11836.
14. Mostrou, S.; Sipöcz, T.; Nagl, A.; Födi, B.; Darvas, F.; Föttinger, K.; van Bokhoven, J. A., Catalytic oxidation of aqueous bioethanol: an efficient upgrade from batch to flow. Reaction Chemistry & Engineering 2018, 3 (5), 781-789.
15. Jorgensen, B.; Egholm Christiansen, S.; Dahl Thomsen, M. L.; Christensen, C. H., Aerobic oxidation of aqueous ethanol using heterogeneous gold catalysts: Efficient routes to acetic acid and ethyl acetate. Journal of Catalysis 2007, 251 (2), 332-337.
16. Zhou, W.; Cheng, K.; Kang, J.; Zhou, C.; Subramanian, V.; Zhang, Q.; Wang, Y., New horizon in C1 chemistry: breaking the selectivity limitation in transformation of syngas and hydrogenation of CO2 into hydrocarbon chemicals and fuels. Chemical Society Reviews 2019, 48 (12), 3193-3228.
17. Dimian, A. C.; Kiss, A. A., Novel energy efficient process for acetic acid production by methanol carbonylation. Chemical Engineering Research and Design 2020, 159, 1-12.
18. Ren, Z.; Lyu, Y.; Song, X.; Ding, Y., Review of heterogeneous methanol carbonylation to acetyl species. Applied Catalysis A: General 2020, 595, 117488.
19. Park, K.; Lim, S.; Baik, J. H.; Kim, H.; Jung, K.-D.; Yoon, S., Exceptionally stable Rh-based molecular catalyst heterogenized on a cationically charged covalent triazine framework support for efficient methanol carbonylation. Catalysis Science & Technology 2018, 8 (11), 2894-2900.
20. Ren, Z.; Lyu, Y.; Song, X.; Liu, Y.; Jiang, Z.; Lin, R.; Ding, Y., Dual-lonically Bound Single-Site Rhodium on Porous Ionic Polymer Rivals Commercial Methanol Carbonylation Catalysts. Advanced Materials 2019, 31 (50), 1904976.
21. Dionisi, D.; Silva, I. M. O., Production of ethanol, organic acids and hydrogen: an opportunity for mixed culture biotechnology? Reviews in Environmental Science and Bio/Technology 2016, 15 (2), 213-242.
22. Knox, W.; Roth, J.; Paulik, F.; Hershman, A. Production of Carboxylic Acids and Esters. U.S. Patent 3769329A
23. Watson, D. J. The CativaTM Process for the Production of Acetic Acid. In Catalysis of Organic Reactions; Herkes, F. E., Ed.; Marcel Dekker: 1998; pp 369-380
24. Nie, X.; Ren, X.; Tu, C.; Song, C.; Guo, X.; Chen, J. G., Computational and experimental identification of strong synergy of the Fe/ZnO catalyst in promoting acetic acid synthesis from CH4 and CO2. Chemical Communications 2020, 56 (28), 3983-3986.
25. Periana, R. A.; Mironov, O.; Taube, D.; Bhalla, G.; Jones, C. J., Catalytic, Oxidative Condensation of CH4 to CH3COOH in One Step via CH Activation. Science 2003, 301 (5634), 814-818.
26. Taniguchi, Y.; Hayashida, T.; Kitamura, T.; Fujiwara, Y., Vanadium-catalyzed acetic acid synthesis from methane and carbon dioxide. In Studies in Surface Science and Catalysis, Inui, T.; Anpo, M.; Izui, K.; Yanagida, S.; Yamaguchi, T., Eds. Elsevier: 1998; Vol. 114, pp 439-442.
27. Huang, W.; Xie, K. C.; Wang, J. P.; Gao, Z. H.; Yin, L. H.; Zhu, Q. M., Possibility of Direct Conversion of CH4 and CO2 to High-Value Products. Journal of Catalysis 2001, 201 (1), 100-104.
28. Wilcox, E. M.; Roberts, G. W.; Spivey, J. J., Direct catalytic formation of acetic acid from CO2 and methane. Catalysis Today 2003, 88 (1), 83-90.
29. H.J. Freund, J. Wambach, Process for the Preparation of Acetic Acid, Republic of South Africa, 7 August 1995, pp. 95-6606
30. Montejo-Valencia, B. D.; Pagán-Torres, Y. J.; Martínez-Iñesta, M. M.; Curet-Arana, M. C., Density Functional Theory (DFT) Study To Unravel the Catalytic Properties of M-Exchanged MFI, (M = Be, Co, Cu, Mg, Mn, Zn) for the Conversion of Methane and Carbon Dioxide to Acetic Acid. ACS Catalysis 2017, 7 (10), 6719-6728.
31. Wang, S.; Guo, S.; Luo, Y.; Qin, Z.; Chen, Y.; Dong, M.; Li, J.; Fan, W.; Wang, J., Direct synthesis of acetic acid from carbon dioxide and methane over Cu-modulated BEA, MFI, MOR and TON zeolites: a density functional theory study. Catalysis Science & Technology 2019, 9 (23), 6613-6626.
32. Rabie, A. M.; Betiha, M. A.; Park, S.-E., Direct synthesis of acetic acid by simultaneous coactivation of methane and CO2 over Cu-exchanged ZSM-5 catalysts. Applied Catalysis B: Environmental 2017, 215, 50-59.
33. Wu, J.-F.; Yu, S.-M.; Wang, W. D.; Fan, Y.-X.; Bai, S.; Zhang, C.-W.; Gao, Q.; Huang, J.; Wang, W., Mechanistic Insight into the Formation of Acetic Acid from the Direct Conversion of Methane and Carbon Dioxide on Zinc-Modified H-ZSM-5 Zeolite. Journal of the American Chemical Society 2013, 135 (36), 13567-13573.
34. Mlekodaj, K.; Dedecek, J.; Pashkova, V.; Tabor, E.; Klein, P.; Urbanova, M.; Karcz, R.; Sazama, P.; Whittleton, S. R.; Thomas, H. M.; Fishchuk, A. V.; Sklenak, S., Al Organization in the SSZ-13 Zeolite. Al Distribution and Extraframework Sites of Divalent Cations. The Journal of Physical Chemistry C 2019, 123 (13), 7968-7987.
35. Patent CZ2017832A (SSZ-13,CHA), patent WO 2015/197036A1
36. Pilar, R.; Moravkova, J.; Sadovska, G.; Sklenak, S.; Brabec, L.; Pastvova, J.; Sazama, P., Controlling the competitive growth of zeolite phases without using an organic structure-directing agent. Synthesis of Al-rich *BEA. Microporous and Mesoporous Materials 2022, 333, 111726.
37. patent WO2020200336-A1 CZ201900210-A3
38. Tarach, K.; Sobalska, J.; Held, A.; Dedecek, J.; Tabor, E.; Góra-Marek, K., Oxygen Splitting and Methane Oxidation over Fe-Mordenite: Insight by the Operando 2D COS UV-Vis-NIR-IR Approach. The Journal of Physical Chemistry C 2024, 128 (9), 3759-3769.
39. Kornas, A.; Tabor, E.; Wierzbicki, D. K.; Olszowka, J. E.; Pilar, R.; Dedecek, J.; Sliwa, M.; Jirglova, H.; Sklenak, S.; Rutkowska-Zbik, D.; Mlekodaj, K., Activation of molecular oxygen over binuclear iron centers in Al-rich *BEA zeolite. Applied Catalysis B: Environmental 2023, 336, 122915.
40. Mlekodaj, K.; Lemishka, M.; Kornas, A.; Wierzbicki, D. K.; Olszowka, J. E.; Jirglová, H.; Dedecek, J.; Tabor, E., Evolution of Active Oxygen Species Originating from O2 Cleavage over Fe-FER for Application in Methane Oxidation. ACS Catalysis 2023, 13 (5), 3345-3355.
41. Dedecek, J.; Tabor, E.; Andrikopoulos, P. C.; Sklenak, S., Splitting dioxygen over distant binuclear transition metal cationic sites in zeolites. Effect of the transition metal cation. International Journal of Quantum Chemistry 2021, 121 (10), e26611.
42. Mlekodaj, K.; Lemishka, M.; Sklenak, S.; Dedecek, J.; Tabor, E., Dioxygen splitting at room temperature over distant binuclear transition metal centers in zeolites for direct oxidation of methane to methanol. Chemical Communications 2021, 57 (28), 3472-3475.
43. Tabor, E.; Lemishka, M.; Olszowka, J. E.; Mlekodaj, K.; Dedecek, J.; Andrikopoulos, P. C.; Sklenak, S., Splitting Dioxygen over Distant Binuclear Fe Sites in Zeolites. Effect of the Local Arrangement and Framework Topology. ACS Catalysis 2021, 11 (4), 2340-2355.
44. Tabor, E.; Dedecek, J.; Mlekodaj, K.; Sobalik, Z.; Andrikopoulos, P. C.; Sklenak, S., Dioxygen dissociation over man-made system at room temperature to form the active α-oxygen for methane oxidation. Science Advances 6 (20), eaaz9776.
45. Sobalik, Z.; Novakova, J.; Dedecek, J.; Sathu, N. K.; Tabor, E.; Sazama, P.; Stastny, P.; Wichterlova, B., Tailoring of Fe-ferrierite for N2O decomposition: On the decisive role of framework Al distribution for catalytic activity of Fe species in Fe-ferrierite. Microporous and Mesoporous Materials 2011, 146 (1), 172-183.
46. M. Bourgogne, J.-L Guth, R. Wey, patent USA 4 503 024 (1985
47. Mingos, D. M. P.; Duan, X.; L., H.; Gade; Lu, Y.; Parkin, G.; Neese, F.; Pariente, J. P.; Poeppelmeier, K. R.; S.; Schneider; Armstrong, F. A.; Stalke, D.; M.; Takano, Insights into the Chemistry of Organic Structure-Directing Agents in the Synthesis of Zeolitic Materials. Insights into the Chemistry of Organic Structure-Directing Agents in the Synthesis of Zeolitic Materials 2018**.**
48. Calligaris, M.; Nardin, G.; Randaccio, L., Cation site location in hydrated chabazites. Crystal structure of potassium- and silver- exchanged chabazites. Zeolites 1983, 3 (3), 205-208.
49. CZ2017832A3, https://patents.google.com/patent/CZ2017832A3/cs
50. Mlekodaj, K.; Bernauer, M.; Olszowka, J. E.; Klein, P.; Pashkova, V.; Dedecek, J., Synthesis of the Zeolites from SBU: An SSZ-13 Study. Chemistry of Materials 2021, 33 (5), 1781-1788.
51. patent WO2015/197036A1
52. Tabor, E.; Závěta, K.; Sathu, N. K.; Tvar 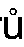 žková, Z.; Sobalík, Z., Characterization of iron cationic sites in ferrierite using Mössbauer spectroscopy. *Catalysis Today* **2011,** *169* (1), 16-23.
53. CZ 293917 B6 (2001)

## Claims

1. A catalyst for the production of acetic acid and methyl acetate from methane and carbon dioxide, **characterized in that** it comprises a zeolite containing:
- Al atoms arranged in pairs, wherein each pair is represented by two Al atoms located in one zeolite ring,
- cations of a divalent transition metal M, preferably Fe and Zn,
wherein the ratio of transition metal M to Al is in the range of 0.06 to 0.45, the weight content of transition metal M is less than 10% by weight, and the Si/Al ratio of the zeolite is in the range of 2.5 to 10,
wherein the zeolite has a structure selected from CHA, FER, MOR, or *BEA structures determined according to the Nickel-Strunz classification of zeolite structures.

2. A method for producing acetic acid and methyl acetate, **characterized in that**:
a) water and ammonium ions are removed from the catalyst, preferably by heating for a period longer than 30 minutes at a temperature above 300 °C, wherein a catalyst according to the invention as defined above is used,
b) the catalyst is contacted first with methane and subsequently with carbon dioxide at temperatures below 450 °C under at least atmospheric pressure (1 atm.).

3. The method according to claim 2, **characterized in that** in step (a) the heating is carried out under vacuum or in a stream of an inert gas, preferably helium or nitrogen.

4. The method according to claim 2, **characterized in that** in step (a) the heating is carried out at a temperature of at least 500 °C.
